(19) Europäisches Patentamt
European Patent Office
Office européen des brevets

(11) **EP 2 196 360 B1**

(12) **EUROPEAN PATENT SPECIFICATION**

(45) Date of publication and mention
of the grant of the patent:
**03.08.2011 Bulletin 2011/31**

(51) Int Cl.:
***B60R 21/0134*** *(2006.01)*    ***A61N 1/36*** *(2006.01)*

(21) Application number: **08425788.0**

(22) Date of filing: **10.12.2008**

(54) **Prevention of physical injury to an occupant of a vehicle due to the whiplash effect**

Vorbeugung der Verletzung von Fahrzeuginsassen infolge eines Peitschenhieb-Effekts

Prévention de blessure physique d'un occupant d'un véhicule due aux effets du coup du lapin

(84) Designated Contracting States:
**DE FR IT TR**

(43) Date of publication of application:
**16.06.2010 Bulletin 2010/24**

(73) Proprietor: **Fiat Group Automobiles S.p.A.**
**10135 Torino (TO) (IT)**

(72) Inventor: **Pascolo, Paolo**
**33100 Udine (IT)**

(74) Representative: **Bergadano, Mirko et al**
**Studio Torta S.r.l.**
**Via Viotti, 9**
**10121 Torino (IT)**

(56) References cited:
**EP-A- 1 958 842**    **WO-A-03/049976**
**US-A1- 2001 040 065**    **US-A1- 2006 031 015**

**Description**

The present invention relates to the prevention of physical injury to an occupant of a vehicle due to the whiplash effect.

Electrostimulation devices are known that are able to prevent injury and/or trauma to the occupant as a result of the "whiplash" effect which said occupant suffers as a consequence of a violent counter-blow due to a sudden and unexpected acceleration or deceleration of the vehicle, for instance in the case of an impact in an accident.

For example, international patent application WO 03/049976 describes a system according to the preamble of claim 1 comprising a sensory stimulation device, in particular an electrostimulation device, which is installed on board a vehicle and basically comprises sensors that detect anomalous variations in particular parameters of the conditions of use of the vehicle; electrodes that are able to generate electrical signals to stimulate parts of the user's body, in particular the paravertebral muscles; and an electronic control unit able to detect, on the basis of said anomalous variations, the impact of the vehicle, in order to activate the electrodes, upon detection of the impact, to generate the electrical stimulation signals which conveniently cause localized contractions of the muscles of the occupant's body.

Although the electrostimulation devices described above are quite efficient the need is felt to improve the effect of the sensory stimulation device on the occupant, in order to further reduce and/or completely eliminate traumatic injury to the body of the occupant due to the whiplash effect.

US2006/0031015 relates to a process of determining an imminent-collision between a vehicle and an object, said vehicle having a sensing system for obtaining one or more images representing at least one observed object within a field of detection, said process comprising: (a) obtaining one or more images representing an observed object within said field of detection; and (b) determining that a collision between said vehicle and said observed object is imminent when the ratio of the probability that said observed object is actually within a collision zone to the probability that said observed object is actually within a safe zone is greater than a certain value and deploying collision mitigation measures accordingly.

This need is met by the present invention, which relates to a system for preventing physical injury to an occupant of a vehicle due to the whiplash effect as set forth in the appended claims.

According to the present invention there is also provided a vehicle fitted with a system for preventing physical injury and/or trauma to an occupant of a vehicle according to that claimed in claim 12.

In order to better understand the present invention, a non-limiting preferred embodiment thereof will now be described by way of example with reference to the accompanying drawings, in which:

- figure 1 is a schematic diagram of a terrestrial vehicle provided with a system for preventing physical injury to an occupant of the vehicle due to the whiplash effect, produced according to the present invention; while
- figure 2 is a flow diagram of the procedures implemented by the system illustrated in figure 1.

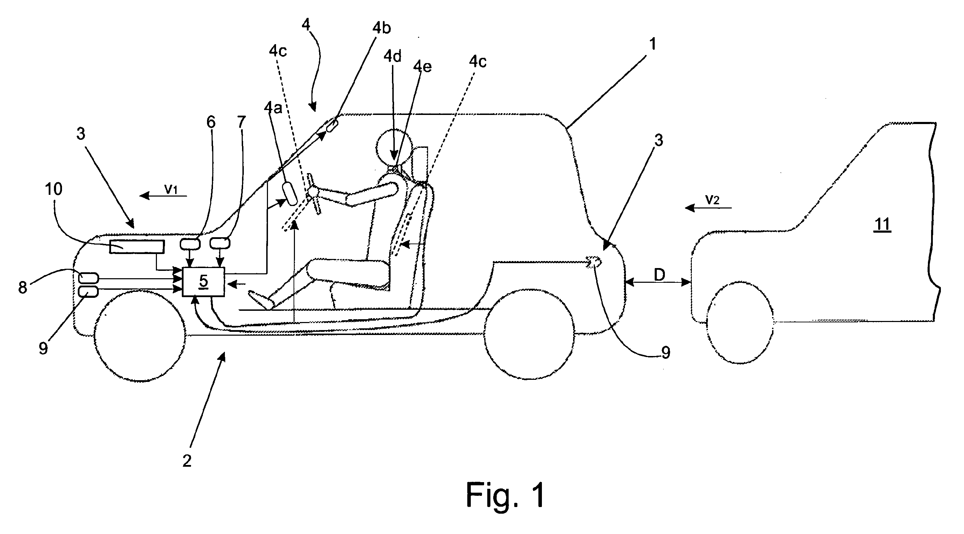

With reference to figure 1, designated as a whole with number 1 is a vehicle, corresponding to a terrestrial vehicle, comprising a system 2 for preventing physical injury to an occupant of the vehicle 1 due to the whiplash effect.

However it should be noted that the vehicle 1 could be an aircraft or a boat or any other similar means of transport.

The system 2 is able to prevent the so-called whiplash effect on an occupant of the vehicle in the event of an impact.

The system 2 essentially comprises a sensor system 3 to measure the monitoring values associated with an impact condition of the vehicle 1; a sensory stimulation device 4 able to cause, when activated, a complete localised contraction of the muscles of the occupant's body; and an electronic control unit 5, configured to process the monitoring values measured by the sensor system 3 in order to predict the time of impact of the vehicle $t_u$ a certain amount of time in advance.

The sensory stimulation device 4 is able to generate stimulation signals towards the occupant so as to cause a complete localised contraction of the muscles of the body thereof.

The sensory stimulation device 4 comprises a combination of one or more from among several devices to stimulate the occupant's senses, for instance: an acoustic stimulation device 4a that generates one or more acoustic signals towards the occupant; and/or a visual stimulation device 4b able to generate one or more light signals towards the occupant suitable to cause a reaction resulting in the contraction of the muscles thereof. Additional stimulation devices may include a tactile stimulation device 4c able to generate signals corresponding to mechanical vibrations preferably, but not necessarily, on the steering wheel and/or on the seat of the vehicle suitable to cause a contraction of the muscles of the body of the occupant; and or an electrical stimulation device 4d provided with stimulation electrodes 4e able to generate electrical signals to be applied to predefined parts of the occupant's body.

Once the time of impact of the vehicle $t_u$ has been determined, the electronic control unit 5 activates the sensory stimulation device 4 so that it generates stimulation signals at a point in time $t_i$, which precedes said time of impact of the vehicle $t_u$ by a set amount of time $\Delta t$, so that the effect of the stimulation of the muscles of the occupant is wholly complete by the time said impact occurs.

In other words, the electronic control unit 5 is able to predict the time of impact of the vehicle $t_u$ in order to stimulate the occupant a set amount of time in advance $\Delta t$ with respect to the time of impact of the vehicle $t_u$ in order to achieve the complete localized contraction of the muscles of the occupant's body at the time of impact of the vehicle 1.

With reference to the example illustrated in figure 1, the stimulation electrodes 4e of the electric stimulation device 4d are produced as described and illustrated in international patent application WO03/049976 and are not therefore described in further detail, except to specify that they may be placed directly on the occupant's body in order to electrostimulate the muscles, preferably, but not necessarily, the paravertebral muscles, or they may be inserted into a collar and/or in the rear padding of a safety helmet (not illustrated), and/or in a seat or in soft, retractable fork-shaped supports in the head-rest of the vehicle 1 and/or on the arms of the occupant's spectacles or on any other similar item that can be worn by said occupant and that can arranged so as to come at least partially in contact with the user's body.

The sensor system 3 comprises a sensor 6 for measuring speed $v_1$ and/or a sensor 7 for measuring the inclination of the vehicle 1 with respect to its bearing plane, such as for instance an inclinometer; and/or a sensor 8 for measuring the acceleration, for instance an accelerometer.

The sensor system 3 may also comprise devices 9 for measuring the distance D between the vehicle 1 and other vehicles 11 in an area around it, such as for example radars and/or ultrasound sensors or similar sensors installed on board the vehicle 1.

The sensor system 3 may also comprise, preferably, but not necessarily, a vehicle signalling system 10 through which the electronic control unit 5 receives information from the electronic control units installed on board the other vehicles 11 concerning the movements of said vehicles 11. In particular the vehicle signalling system 10 can comprise a wireless communication system, preferably, but not necessarily, a Bluetooth system, through which the electronic control unit 5 of the vehicle 1 receives data relating to the speed $v_2$ and/or the acceleration and/or the distance of a vehicle 11 when the latter is within a certain range of action with respect to said vehicle 1.

The electronic control unit 5 receives from the sensor system 3 the monitoring values associated with an impact condition of the vehicle 1 or the speed $v_1$ and/or the acceleration and/or the inclination of the vehicle 1, and/or the speed $v_2$ of the vehicles present in correspondence with said vehicle 1, and/or the distance D of the vehicle 1 from obstacles or vehicles 11 present in correspondence with said vehicle 1.

The electronic control unit 5 is able to process said monitoring values in order to estimate the time $t_u$ of impact of the vehicle 1 in advance.

In particular, the electronic control unit 5 implements an estimation algorithm that determines the time of impact $t_u$ of the vehicle 1, on the basis of the differential in speed $v_1 - v_2$ between the vehicle 1 and the vehicles 11 present in an area around said vehicle 1, and/or as a function of the distance D between the vehicle 1 and the vehicles 11.

The electronic control unit 5. is also able to determine an ideal point in time $t_i$ for activating the sensory stimulation device 4, as a function of the time of impact of the vehicle $t_u$ and of an set time in advance $\Delta t$. In the specific case, the ideal point in time for activating the stimulation electrodes $t_i$ is calculated using the following ratio:

$$t_i = t_u - \Delta t;$$

The time in advance $\Delta t$ is between approximately 50 ms and 200 ms.

It is important to note that laboratory tests have demonstrated that stimulation of the muscles of the human body, in particular electrical stimulation, performed within a range $\Delta t$ of between approximately 50-200 ms in advance with respect to the time of impact of the vehicle $t_u$, conveniently allows for compensation of the intrinsic circuit delays introduced in the electrical signals by the electronic circuits/electronic components used in the electrostimulation system, and the response times of the occupant's body to the electrostimulation associated with controlling balance (vestibular system) and/or spinal control (reflex system) by the user.

The electronic control unit 5 is also able to activate the sensory stimulation device 4 in order to generate the stimulation signal at the point in time $t_i$. In that connection, the electronic control unit 5, can generate a command signal at the point in time $t_i$ that activates the sensory stimulation device 4.

The electronic control unit 5 can be connected to the devices 4a, 4b, 4c and 4d of the sensory stimulation device 4 by means of relative cables and connectors as shown in figure 1, or could be able to transmit the command signal to said devices 4a, 4b, 4c and 4d of the sensory stimulation device 4 by means of a wireless communication system such as for instance a communication system using Bluetooth protocol.

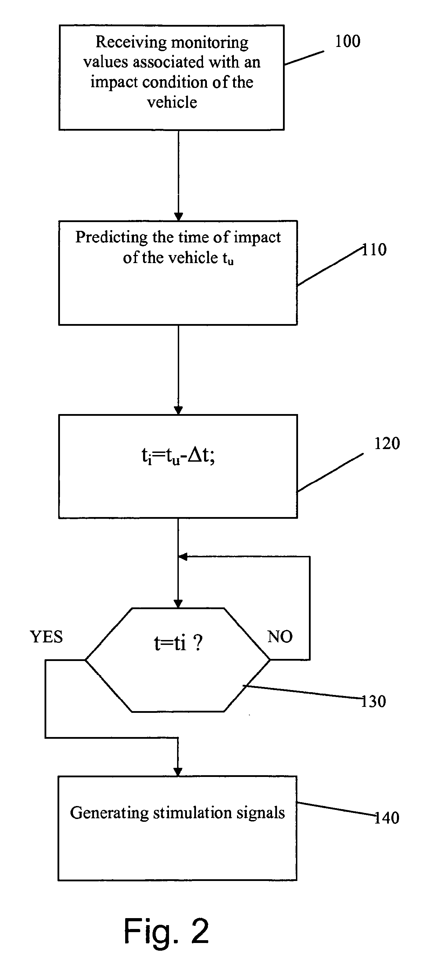

With reference to figure 2, in use, the electronic control unit 5 of the system 2 receives instant by instant the monitoring values associated with an impact condition of the vehicle 1 from the sensor system 3 (block 100).

At this point the electronic control unit 5 implements the estimation algorithm to process the monitoring values associated with an impact condition of the vehicle 1 in order to predict the time of impact of the vehicle $t_u$ (block 110).

When the time of impact $t_u$ has been estimated, the electronic control unit 5 determines the ideal point in time $t_i$ for activating the sensory stimulation device 4 (block 120) using the ratio described above.

At this point the electronic control unit 5 can start a timing procedure, for example by means of an internal clock, and at the point in time $t_i$ (YES output from block 130) it generates the command signal that activates the acoustic stimulation device 4a, and/or the visual stimulation device 4b.

The system described above is extremely advantageous in that thanks to the preventive activation in advance of the sensory stimulation device the effect of stimulation on the muscles of the user's body is advantageously complete when the impact occurs, significantly reducing the trauma or physical injury to the occupant due to the whiplash effect.

**Claims**

1. System for preventing physical injury and/or trauma to an occupant of a vehicle (2), comprising:

   • sensor means (3) configured to monitor values so as to enable the determination of an impact condition of the vehicle (1); and
   • sensory stimulation means (4) configured to generate stimulation signals such as to cause a localised contraction of the muscles of the body of said occupant on the basis of the monitored values;

   said system being **characterized in that** said sensory stimulation means (4) comprise acoustic stimulation means (4a) that generate acoustic stimulation signals towards the occupant, and/or visual stimulation means (4b) that generate light stimulation signals towards the occupant, and said system comprises an electronic control unit (5) so that said sensory stimulation means (4) and the electronic control unit 5) are configured to:

   • predict a time of impact of the vehicle (tu) as a function of the measured values; and
   • generate said stimulation signals, between approximately 50 ms and 200 ms in advance with respect to said time of impact of the vehicle (tu) so as to cause a complete localised contraction of the muscles of the body by the time said impact occurs.

2. System according to Claim 1, wherein said sensory stimulation means (4) comprise: tactile stimulation means (4c) that generate mechanical vibrations perceivable by the occupant of said vehicle (1), and/or electrical stimulation means (4d) that generate electrical stimulation signals to apply to predefined parts of said occupant's body.

3. System according to Claim 2, wherein said electrical stimulation means (4d) comprise at least one stimulation electrode (4e).

4. System according to Claim 3, wherein said sensory stimulation means (4) comprise processing means (5) suitable to control said stimulation electrode (4e) in order to generate said electrical stimulation signals.

5. System according to any one of the previous Claims, wherein said sensor means (3) comprise devices for measuring the distance (9) of said vehicle (1) with respect to other vehicles (11).

6. System according to any one of the previous Claims, wherein said sensor means (3) comprise a sensor for measuring speed (6), and/or a sensor for measuring the inclination of the vehicle (7) with respect to its bearing plane, and/or a sensor (8) for measuring the acceleration.

7. System according to any one of the previous Claims, wherein said sensor means (3) comprise a vehicle signalling system (10) able to implement a wireless communication between said processing means (5) installed on board said vehicle (1) and processing means installed on board at least one other vehicle (11) to receive information about the movements of said vehicle (11).

8. System according to any one of the previous Claims, wherein said processing means (5) are connected to said sensory stimulation means (4) by means of relative cables and connectors.

9. System according to any one of the previous claims, wherein said processing means (5) are able to control said sensory stimulation means (4) by means of a wireless communication system.

10. System according to any one of the Claims from 3 to 9, wherein said stimulation electrode (4) is able to be applied to at least one localised part of the body of said user to transmit at least one electrical impulse to said part.

11. System according to any one of the Claims from 4 to 10, wherein said electrical stimulation means (4) are inserted into the rear padding of a safety helmet, and/or in a collar, and/or in a seat or in soft, retractable fork-shaped supports in the head-rest of said means of transport and/or on the arms of the occupant's spectacles.

12. Vehicle (1) comprising a system for preventing injury and/or trauma to at least one occupant of the vehicle (2) according to any one of the previous Claims.

**Patentansprüche**

1. System zur Vorbeugung einer Verletzung und/oder eines Traumas eines Fahrzeuginsassen (2), welches umfasst:

   • Sensormittel (3), die dafür ausgebildet sind, Werte zu überwachen, um die Bestimmung eines Aufprallzustands des Fahrzeugs (1) zu ermöglichen; und
   • Mittel zur sensorischen Stimulation (4), die dafür ausgebildet sind, Stimulationssignale zu erzeugen, derart, dass eine lokal begrenzte Kontraktion der Muskeln des Körpers des Insassen auf der Basis der überwachten Werte verursacht wird;

   wobei das System **dadurch gekennzeichnet ist, dass** die Mittel zur sensorischen Stimulation (4) Mittel zur akustischen Stimulation (4a), welche auf den Insassen einwirkende akustische Stimulationssignale erzeugen, und/oder Mittel zur visuellen Stimulation (4b), welche auf den Insassen einwirkende Lichtstimulationssignale erzeugen, umfassen, und das System eine elektronische Steuereinheit (5) umfasst, so dass die Mittel zur sensorischen Stimulation (4) und die elektronische Steuereinheit (5) dafür ausgebildet sind,

   • einen Aufprallzeitpunkt des Fahrzeugs (tu) in Abhängigkeit von den gemessenen Werten vorherzusagen; und
   • die Stimulationssignale zwischen etwa 50 ms und 200 ms im Voraus, bezogen auf den Aufprallzeitpunkt des Fahrzeugs (tu), zu erzeugen, so dass bis zu dem Zeitpunkt, zu dem der Aufprall erfolgt, eine vollständige, lokal begrenzte Kontraktion der Muskeln des Körpers bewirkt wird.

2. System nach Anspruch 1, wobei die Mittel zur sensorischen Stimulation (4) umfassen: Mittel zur taktilen Stimulation (4c), welche mechanische Schwingungen erzeugen, die von dem Insassen des Fahrzeugs (1) wahrnehmbar sind, und/oder Mittel zur elektrischen Stimulation (4d), welche elektrische Stimulationssignale erzeugen, die an vordefinierte Körperteile des Insassen anzulegen sind.

3. System nach Anspruch 2, wobei die Mittel zur elektrischen Stimulation (4d) mindestens eine Stimulationselektrode (4e) umfassen.

4. System nach Anspruch 3, wobei die Mittel zur sensorischen Stimulation (4) Verarbeitungsmittel (5) umfassen, die geeignet sind, die Stimulationselektrode (4e) zu steuern, um die elektrischen Stimulationssignale zu erzeugen.

5. System nach einem der vorhergehenden Ansprüche, wobei die Sensormittel (3) Vorrichtungen zum Messen des Abstands (9) des Fahrzeugs (1) in Bezug auf andere Fahrzeuge (11) umfassen.

6. System nach einem der vorhergehenden Ansprüche, wobei die Sensormittel (3) einen Sensor zum Messen der Geschwindigkeit (6) und/oder einen Sensor (7) zum Messen der Neigung des Fahrzeugs in Bezug auf seine Standfläche und/oder einen Sensor (8) zum Messen der Beschleunigung umfassen.

7. System nach einem der vorhergehenden Ansprüche, wobei die Sensormittel (3) ein Fahrzeug-Signalisierungssystem (10) umfassen, das in der Lage ist, eine drahtlose Kommunikation zwischen den Verarbeitungsmitteln (5), die an Bord des Fahrzeugs (1) installiert sind, und Verarbeitungsmitteln, die an Bord mindestens eines weiteren Fahrzeugs (11) installiert sind, zu implementieren, um Informationen über die Bewegungen des Fahrzeugs (11) zu empfangen.

8. System nach einem der vorhergehenden Ansprüche, wobei die Verarbeitungsmittel (5) mit den Mitteln zur sensorischen Stimulation (4) mittels entsprechender Kabel und Verbinder verbunden sind.

9. System nach einem der vorhergehenden Ansprüche, wobei die Verarbeitungsmittel (5) in der Lage sind, die Mittel zur sensorischen Stimulation (4) mittels eines drahtlosen Kommunikationssystems zu steuern.

10. System nach einem der Ansprüche 3 bis 9, wobei die Stimulationselektrode (4) geeignet ist, an mindestens einen lokal begrenzten Körperteil des Benutzers angelegt zu werden, um mindestens einen Impuls zu diesem Körperteil zu übertragen.

11. System nach einem der Ansprüche 4 bis 10, wobei die Mittel zur elektrischen Stimulation (4) in die hintere Polsterung eines Schutzhelms und/oder in einen Kragen und/oder in einen Sitz oder in weiche, zurückziehbare gabelförmige Abstützungen in der Kopfstütze des Transportmittels eingesetzt und/oder an den Bügeln der Brille des Insassen angebracht sind.

12. Fahrzeug (1), welches ein System zur Vorbeugung einer Verletzung und/oder eines Traumas mindestens eines Fahrzeuginsassen (2) nach einem der vorhergehenden Ansprüche umfasst.

**Revendications**

1. Système pour éviter une blessure physique et/ou un traumatisme à un occupant d'un véhicule (2), comprenant :

• des moyens de capteur (3) configurés pour surveiller des valeurs afin de permettre la détermination d'une condition d'impact du véhicule (1) ; et
• des moyens de stimulation sensorielle (4) configurés pour générer des signaux de stimulation de manière à entraîner une contraction localisée des muscles du corps dudit occupant sur la base des valeurs surveillées ;

ledit système étant **caractérisé en ce que** lesdits moyens de stimulation sensorielle (4) comprennent des moyens de stimulation acoustique (4a) qui génèrent des signaux de stimulation acoustique vers l'occupant, et/ou des moyens de stimulation visuelle (4b) qui génèrent des signaux de stimulation lumineuse vers l'occupant, et ledit système comprend une unité de commande électronique (5) de sorte que lesdits moyens de stimulation sensorielle (4) et l'unité de commande électronique (5) sont configurés pour :

• prédire un instant d'impact du véhicule (tu) en fonction des valeurs mesurées et
• générer lesdits signaux de stimulation, entre approximativement 50 ms et 200 ms en avance par rapport audit instant d'impact du véhicule (tu) de manière à entraîner une contraction localisée complète des muscles du corps à l'instant où ledit impact se produit.

2. Système selon la revendication 1, dans lequel lesdits moyens de stimulation sensorielle (4) comprennent : des moyens de stimulation tactile (4c) qui génèrent des vibrations mécaniques perceptibles par l'occupant dudit véhicule (1), et/ou des moyens de stimulation électrique (4d) qui génèrent des signaux de stimulation électriques à appliquer à des parties prédéfinies du corps dudit occupant.

3. Système selon la revendication 2, dans lequel lesdits moyens de stimulation électrique (4d) comprennent au moins une électrode de stimulation (4e).

4. Système selon la revendication 3, dans lequel lesdits moyens de stimulation sensorielle (4) comprennent des moyens de traitement (5) adaptés pour commander ladite électrode de stimulation (4e) afin de générer lesdits signaux de stimulation électrique.

5. Système selon l'une quelconque des revendications précédentes, dans lequel lesdits moyens de capteur (3) comprennent des dispositifs pour mesurer la distance (9) dudit véhicule (1) par rapport à d'autres véhicules (11).

6. Système selon l'une quelconque des revendications précédentes, dans lequel lesdits moyens de capteur (3) comprennent un capteur pour mesurer la vitesse (6), et/ou un capteur pour mesurer l'inclinaison du véhicule (7) par rapport à son plan d'appui, et/ou un capteur (8) pour mesurer l'accélération.

7. Système selon l'une quelconque des revendications précédentes, dans lequel lesdits moyens de capteur (3) comprennent un système de signalisation de véhicule (10) capables de mettre en oeuvre une communication sans fil entre lesdits moyens de traitement (5) installés à bord dudit véhicule (1) et des moyens de traitement installés à bord d'au moins un autre véhicule (11) pour recevoir des informations sur les mouvements dudit véhicule (11).

8. Système selon l'une quelconque des revendications précédentes dans lequel lesdits moyens de traitement (5) sont reliés auxdits moyens de stimulation sensorielle (4) au moyen de câbles et de connecteurs relatifs.

9. Système selon l'une quelconque des revendications précédentes, dans lequel lesdits moyens de traitement (5) peuvent commander lesdits moyens de stimulation sensorielle (4) au moyen d'un système de communication sans fil.

10. Système selon l'une quelconque des revendications 3 à 9, dans lequel ladite électrode de stimulation (4e) peut être appliquée sur au moins une partie localisée du corps dudit utilisateur pour transmettre au moins une impulsion électrique à ladite partie.

11. Système selon l'une quelconque des revendications 4 à 10 dans lequel lesdits moyens de stimulation électrique (4) sont insérés dans le rembourrage arrière d'un casque de sécurité, et/ou dans un collier, et/ou dans un siège ou dans des supports en forme de fourche rétractable, souples dans l'appui-tête desdits moyens de transport et/ou sur les branches des lunettes de l'occupant.

12. Véhicule (1) comprenant un système pour éviter une blessure et/ou un traumatisme à moins un occupant du véhicule (2) selon l'une quelconque des revendications précédentes.

Fig. 1

EP 2 196 360 B1

Receiving monitoring
values associated with an
impact condition of the
vehicle

100

Predicting the time of impact
of the vehicle $t_u$

110

$t_i = t_u - \Delta t;$

120

YES    t=ti ?    NO

130

Generating stimulation signals

140

# Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03049976 A

- US 20060031015 A